# EUROPÄISCHE PATENTANMELDUNG

(11) **EP 4 649 968 A1**
(43) Veröffentlichungstag der Anmeldung: **19.11.2025**
(21) Anmeldenummer: 25171613.0
(22) Anmeldetag: 22.04.2025
(51) Int. Cl.: A61L 2/04, A61L 2/16, A61L 2/22, A61L 2/24, B67C 7/00, B05B 14/40, B05B 16/20

(54) **THERMISCHE BEHÄLTERBEHANDLUNGSVORRICHTUNG UND VERFAHREN ZUM BETREIBEN DER THERMISCHEN BEHÄLTERBEHANDLUNGSVORRICHTUNG**

(30) Priorität: 15.05.2024 DE 102024113557
(71) Anmelder: Krones AG, 93073 Neutraubling (DE)
(72) Erfinder: Kaatz, Stefan, 93073 Neutraubling (DE); Schmuck, Jesse, 93073 Neutraubling (DE)
(74) Vertreter: Grünecker Patent- und Rechtsanwälte PartG mbB

(57) **Zusammenfassung**

Die Erfindung betrifft eine thermische Behälterbehandlungsvorrichtung umfassend eine Fördervorrichtung (45) für Behälter (44), eine Behandlungszone (1, 15, 17, 32-43) mit einer Spritzvorrichtung (2, 17, 18, 58-69) zum Ausbringen von Behandlungsmedium auf Behälter (44), ein Becken (4, 19, 20, 46-57) zum Auffangen und Bereitstellen von Behandlungsmedium und eine Leitungsverbindung (6, 21, 22) zwischen Becken und Spritzvorrichtung, wobei in der Leitungsverbindung eine Pumpe (7, 23, 24) vorgesehen ist. Im Becken ist unterhalb eines Füllniveaus des Behandlungsmediums ein Temperatursensor (8, 25, 26, 84) vorgesehen. Die thermische Behälterbehandlungsvorrichtung umfasst eine Steuerungsvorrichtung (9), die ausgebildet ist, während eines Normalbetriebs in der Behandlungszone die Pumpe mit einer Nennleistung zum Ausbringen von Behandlungsmedium mittels der Spritzvorrichtung zu betreiben und während eines Stand-By-Betriebs in der Behandlungszone die Pumpe nicht zu betreiben oder die Pumpe mit einer reduzierten Nennleistung zu betreiben, ohne dass Behandlungsmedium mittels der Spritzvorrichtung ausbringbar ist. Weiter betrifft die Erfindung ein Verfahren zum Regeln der thermischen Behälterbehandlungsvorrichtung.

## Beschreibung

Die Erfindung betrifft eine thermische Behälterbehandlungsvorrichtung und ein Verfahren zum Betreiben der thermischen Behälterbehandlungsvorrichtung gemäß den unabhängigen Ansprüchen.

### Stand der Technik

Es sind thermische Behälterbehandlungsvorrichtungen, wie Kühler, Wärmer oder Pasteur, bekannt, in denen Behälter in verschiedenen, aufeinanderfolgenden Behandlungszonen mit Behandlungsmedium, das von Spritzvorrichtungen ausgebracht wird, besprüht werden können. Das Ausbringen des Behandlungsmediums durch die Spritzvorrichtungen erfolgt in einem wartenden oder vorbereitenden Betriebsmodus (Stand-By-Modus) ebenso wie in einem normalen Betriebsmodus der thermischen Behälterbehandlungsvorrichtungen. Dies kann hinsichtlich des elektrischen und thermischen Energieverbrauchs als ungünstig angesehen werden.

### Aufgabe

Die Aufgabe der Erfindung ist es, eine thermische Behälterbehandlungsvorrichtung und ein Verfahren zum Betreiben der thermischen Behälterbehandlungsvorrichtung zur Verfügung zu stellen, die eine Einsparung des elektrischen und thermischen Energieverbrauchs und dabei auch eine sofortige Einsatzbereitschaft der thermischen Behälterbehandlungsvorrichtung ermöglichen können.

### Lösung

Die Aufgabe wird gelöst durch die thermische Behälterbehandlungsvorrichtung und das Verfahren zum Betreiben der thermischen Behälterbehandlungsvorrichtung gemäß den unabhängigen Ansprüchen. Weitere Ausführungsformen sind in den Unteransprüchen offenbart.

Die erfindungsgemäße thermische Behälterbehandlungsvorrichtung, wie ein Kühler, ein Wärmer oder ein Pasteur, umfasst eine Fördervorrichtung für Behälter, eine Behandlungszone mit einer Spritzvorrichtung zum Ausbringen von Behandlungsmedium auf Behälter, ein Becken zum Auffangen und Bereitstellen von Behandlungsmedium und eine Leitungsverbindung zwischen Becken und Spritzvorrichtung, wobei in der Leitungsverbindung eine Pumpe vorgesehen ist. Ein Temperatursensor zur Messung einer Temperatur des Behandlungsmediums ist vorgesehen. Die thermische Behälterbehandlungsvorrichtung umfasst weiter eine Steuerungsvorrichtung, die ausgebildet ist, während eines Normalbetriebs in der Behandlungszone die Pumpe mit einer Nennleistung zum Ausbringen von Behandlungsmedium mittels der Spritzvorrichtung zu betreiben und während eines Stand-By-Betriebs in der Behandlungszone die Pumpe nicht zu betreiben oder die Pumpe mit einer reduzierten Nennleistung zu betreiben, ohne dass Behandlungsmedium mittels der Spritzvorrichtung ausbringbar ist.

Durch das Nicht-Betreiben der Pumpe oder durch das Betreiben der Pumpe mit einer reduzierten Nennleistung kann ein thermischer Energieverbrauch reduziert werden, da es zu keiner Zerstäubung des Behandlungsmediums beim Ausbringen mittels der Spritzvorrichtung kommen kann. Auch der elektrische Energieverbrauch durch die Pumpe kann reduziert werden.

Die Temperatur des im Becken vorhandenen Behandlungsmediums kann durch den Temperatursensor gemessen und überwacht werden. So kann beispielsweise die Einsatzbereitschaft der thermischen Behälterbehandlungsvorrichtung nach einem Stand-By-Betrieb ohne größere Verzögerungen gewährleistet werden.

Das Nicht-Betreiben der Pumpe während eines Stand-By-Betriebs kann umfassen, dass die Steuerungsvorrichtung die Pumpe ausgehend von der Nennleistung ausgeschaltet hat. Für einen Übergang in den Normalbetrieb kann die Steuervorrichtung die Pumpe wieder anschalten und mit der Nennleistung betreiben.

Die Leitungsverbindung kann im Becken mit einem Saugstutzen enden und der Temperatursensor kann benachbart zu dem Saugstutzen angeordnet sein.

Ein Bypass kann zwischen der Leitungsverbindung und dem Becken vorgesehen sein, wobei der Bypass oberhalb des Füllniveaus des Behandlungsmediums im Becken enden kann. Beispielsweise kann der Bypass der Pumpe nachfolgend angeordnet sein. Beispielsweise kann in der Leitungsverbindung zwischen der Pumpe und der Spritzvorrichtung eine Klappe vorgesehen sein. Beispielsweise kann der Bypass als ein Rohr mit einem Durchmesser kleiner als 50 Millimeter vorgesehen sein.

Beim Betreiben der Pumpe mit einer reduzierten Nennleistung während des Stand-By-Betriebs können der Bypass und die Klappe in der Leitungsverbindung beispielsweise genutzt werden. Die Klappe kann derart vorgesehen sein, dass Behandlungsmedium, das mittels der mit reduzierter Nennleistung betriebenen Pumpe aus dem Becken abgepumpt werden kann, die Klappe nicht passieren kann, sondern in den Bypass umgelenkt werden kann. Die Höhe der "derart reduzierten Leistung" kann von der verwendeten Klappe und/oder einem Durchmesser des Bypasses abhängen.

Beim Betreiben der Pumpe mit der Nennleistung kann die Klappe hingegen von dem Behandlungsmedium passiert werden. Eine Umlenkung von Behandlungsmedium in den Bypass kann beim Betreiben der Pumpe mit der Nennleistung minimal sein oder ganz unterbunden sein.

Die Leitungsverbindung kann zudem eine Verbindung zu mindestens einem Temperierungsventil aufweisen, wobei das mindestens eine Temperierungsventil mit einem Heiz- und/oder Kühlsystem verbunden sein kann für eine Bereitstellung von temperiertem Behandlungsmedium.

Das mindestens eine Temperierungsventil kann zwischen einem Auslass der Leitungsverbindung aus dem Becken und der Pumpe mit der Leitungsverbindung verbunden sein.

Die Steuerungsvorrichtung kann weiter ausgebildet sein, durch ein Steuern des mindestens einen Temperierungsventils eine Temperatur des Behandlungsmediums im Becken zu steuern.

Die thermische Behälterbehandlungsvorrichtung kann mehrere der Behandlungszonen umfassen, wobei die Behandlungszonen als Aufwärmzonen oder als Abkühlzonen ausgebildet sein können. Beispielsweise können für einen Kühler mehrere aufeinanderfolgende Abkühlzonen vorgesehen sein. Beispielsweise können für einen Wärmer mehrere aufeinanderfolgende Aufwärmzonen vorgesehen sein.

Die thermische Behälterbehandlungsvorrichtung kann mehrere der Behandlungszonen umfassen, wobei mindestens eine davon als eine Aufwärmzone, mindestens eine davon als eine Pasteurisierungszone und mindestens eine davon als eine Abkühlzone ausgebildet sein kann. Die mindestens eine Aufwärmzone und die mindestens eine Abkühlzone können zusammen als eine Rekuperationsstufe ausgebildet sein. Eine erste Leitungsverbindung kann von einem Becken einer Aufwärmzone zu einer Spritzvorrichtung einer Abkühlzone und eine zweite Leitungsverbindung kann von einem Becken einer Abkühlzone zu einer Spritzvorrichtung einer Aufwärmzone vorgesehen sein. Eine dritte Leitungsverbindung kann von einem Becken einer Pasteurisierungszone zur Spritzvorrichtung dieser Pasteurisierungszone vorgesehen sein.

Die Steuerungsvorrichtung kann weiter ausgebildet sein, während eines Stand-By-Betriebs der thermischen Behälterbehandlungsvorrichtung alle Pumpen nicht zu betreiben oder alle Pumpen mit einer reduzierten Nennleistung zu betreiben zum Nicht-Ausbringen von Behandlungsmedium mittels der jeweiligen Spritzvorrichtung.

Die Steuerungsvorrichtung kann weiter ausgebildet sein, nach einem Stand-by-Betrieb der thermischen Behälterbehandlungsvorrichtung zum Erreichen eines Normalbetriebs, zu einem ersten gegebenen Zeitpunkt vor einem geplanten Zuführen von Behältern zu der mindestens einen Aufwärmzone, die Pumpen der mindestens einen Aufwärmzone und der mindestens einen Abkühlzone mit der Nennleistung zu betreiben zum Ausbringen von Behandlungsmedium aus den Spritzvorrichtungen der mindestens einen Aufwärmzone und der mindestens einen Abkühlzone, und danach zu einem zweiten gegebenen Zeitpunkt vor einem geplanten Zuführen von Behältern aus der mindestens einen Anwärmzone zu der mindestens einen Pasteurisierungszone, die Pumpe der mindestens einen Pasteurisierungszone mit der Nennleistung zu betreiben zum Ausbringen von Behandlungsmedium aus der Spritzvorrichtung der mindestens einen Pasteurisierungszone.

Die Steuerungsvorrichtung kann nach Erreichen des Normalbetriebs der thermischen Behandlungsanlage weiter ausgebildet sein zum Erreichen eines Stand-By-Betriebs, nachdem Behälter die mindestens eine Pasteurisierungszone verlassen haben, die Pumpe der mindestens einen Pasteurisierungszone nicht zu betreiben oder die Pumpe der mindestens einen Pasteurisierungszone mit einer reduzierten Nennleistung zu betreiben zum Nicht-Ausbringen von Behandlungsmedium mittels der Spritzvorrichtung der mindestens einen Pasteurisierungszone, und danach nachdem Behälter die letzte der mindestens einen Abkühlzone verlassen haben, alle Pumpen der mindestens einen Aufwärmzone und der mindestens einen Abkühlzone nicht zu betreiben oder alle Pumpen der mindestens einen Aufwärmzone und der mindestens einen Abkühlzone mit einer reduzierten Nennleistung zu betreiben zum Nicht-Ausbringen von Behandlungsmedium mittels der jeweiligen Spritzvorrichtungen.

Der Temperatursensor kann im Becken unterhalb eines Füllniveaus des Behandlungsmediums vorgesehen sein, oder der Temperatursensor kann in einer rückführenden Leitung vorgesehen sein. Die rückführende Leitung kann vom Becken zu einem Heizsystem und/oder einem Kühlsystem führen. Das Kühlsystem kann als ein erster Wärmetauscher ausgebildet sein, der mit dem ersten Temperierungsventil leitungsverbunden sein kann. Das Heizsystem kann als ein zweiter Wärmetauscher ausgebildet sein, der mit dem zweiten Temperierungsventil leitungsverbunden sein kann.

Ein Verfahren zum Regeln einer thermischen Behälterbehandlungsvorrichtung, wie oben oder weiter unten beschrieben, ist vorgesehen.

Das Verfahren kann während eines Stand-By-Betriebs in der Behandlungszone ein Nicht-Betreiben der Pumpe oder Betreiben der Pumpe mit einer reduzierten Nennleistung, ohne dass Behandlungsmedium mittels der Spritzvorrichtung ausgebracht wird, umfassen.

Das Verfahren kann während eines Normalbetriebs in der Behandlungszone, ein Betreiben der Pumpe mit einer Nennleistung zum Ausbringen von Behandlungsmedium mittels der Spritzvorrichtung umfassen.

Für eine thermische Behälterbehandlungsvorrichtung umfassend mindestens ein Temperierungsventils, kann das Verfahren ein Steuern des mindestens einen Temperierungsventils zum Steuern einer Temperatur des Behandlungsmediums im Becken umfassen.

Für eine thermische Behälterbehandlungsvorrichtung umfassend mehrere der Behandlungszonen, wobei mindestens eine davon als eine Aufwärmzone, mindestens eine davon als eine Pasteurisierungszone und mindestens eine davon als eine Abkühlzone ausgebildet sein ist, wobei die mindestens eine Aufwärmzone und die mindestens eine Abkühlzone zusammen als eine Rekuperationsstufe ausgebildet sind, wobei eine erste Leitungsverbindung von einem Becken einer Aufwärmzone zu einer Spritzvorrichtung einer Abkühlzone und eine zweite Leitungsverbindung von einem Becken einer Abkühlzone zu einer Spritzvorrichtung einer Aufwärmzone und wobei eine dritte Leitungsverbindung von einem Becken einer Pasteurisierungszone zur Spritzvorrichtung dieser Pasteurisierungszone vorgesehen ist, kann das Verfahren während eines Stand-By-Betriebs der thermischen Behälterbehandlungsvorrichtung, ein Nicht-Betreiben aller Pumpen oder ein Betreiben aller Pumpen mit einer reduzierten Nennleistung zum Nicht-Ausbringen von Behandlungsmedium mittels der jeweiligen Spritzvorrichtung umfassen.

Das Verfahren kann zum Erreichen eines Normalbetriebs der thermischen Behälterbehandlungsvorrichtung nach einem Stand-by-Betrieb der thermischen Behälterbehandlungsvorrichtung umfassen: zu einem ersten gegebenen Zeitpunkt vor einem geplanten Zuführen von Behältern zu der mindestens einen Aufwärmzone, Betreiben der Pumpen der mindestens einen Aufwärmzone und der mindestens einen Abkühlzone mit der Nennleistung zum Ausbringen von Behandlungsmedium aus den Spritzvorrichtungen der mindestens einen Aufwärmzone und der mindestens einen Abkühlzone, und danach zu einem zweiten gegebenen Zeitpunkt vor einem geplanten Zuführen von Behältern aus der mindestens einen Anwärmzone zu der mindestens einen Pasteurisierungszone, betreiben der Pumpe der mindestens einen Pasteurisierungszone mit der Nennleistung zum Ausbringen von Behandlungsmedium aus der Spritzvorrichtung der mindestens einen Pasteurisierungszone.

Das Verfahren kann zum Erreichen eines Stand-By-Betriebs der thermischen Behandlungsanlage nach Erreichen des Normalbetriebs der thermischen Behandlungsanlage umfassen: nachdem Behälter die mindestens eine Pasteurisierungszone verlassen haben, Nicht-Betreiben der Pumpe der mindestens einen Pasteurisierungszone oder Betreiben der Pumpe der mindestens einen Pasteurisierungszone mit einer reduzierten Nennleistung zum Nicht-Ausbringen von Behandlungsmedium mittels der Spritzvorrichtung der mindestens einen Pasteurisierungszone, und danach nachdem Behälter die letzte der mindestens einen Abkühlzone verlassen haben, Nicht-Betreiben aller Pumpen der mindestens einen Aufwärmzone und der mindestens einen Abkühlzone oder Betreiben aller Pumpen der mindestens einen Aufwärmzone und der mindestens einen Abkühlzone mit einer reduzierten Nennleistung zum Nicht-Ausbringen von Behandlungsmedium mittels der jeweiligen Spritzvorrichtungen.

### Kurze Figurenbeschreibung

Die beigefügten Figuren stellen beispielhaft zum besseren Verständnis und zur Veranschaulichung Aspekte und/oder Ausführungsbeispiele der Erfindung dar. Es zeigt:
Figur 1 eine schematische Ansicht einer Behandlungszone im Normalbetrieb,
Figur 2 die Behandlungszone der Figur 1 im Stand-By-Betrieb,
Figur 3 eine schematische Ansicht zweier Behandlungszonen, die als Rekuperationsstufe geschaltet sind, im Normalbetrieb,
Figur 4 die zwei Behandlungszonen der Figur 3 im Stand-By-Betrieb,
Figur 5 eine schematische Ansicht eines Pasteurs mit Aufwärm-, Pasteurisierungs- und Abkühlzonen im Normalbetrieb,
Figur 6 den Pasteur der Figur 5 im Stand-By-Betrieb,
Figur 7 den Pasteur der Figur 5 beim Einlaufen von Behältern und
Figur 8 den Pasteur der Figur 5 beim Auslaufen von Behältern.

### Ausführliche Figurenbeschreibung

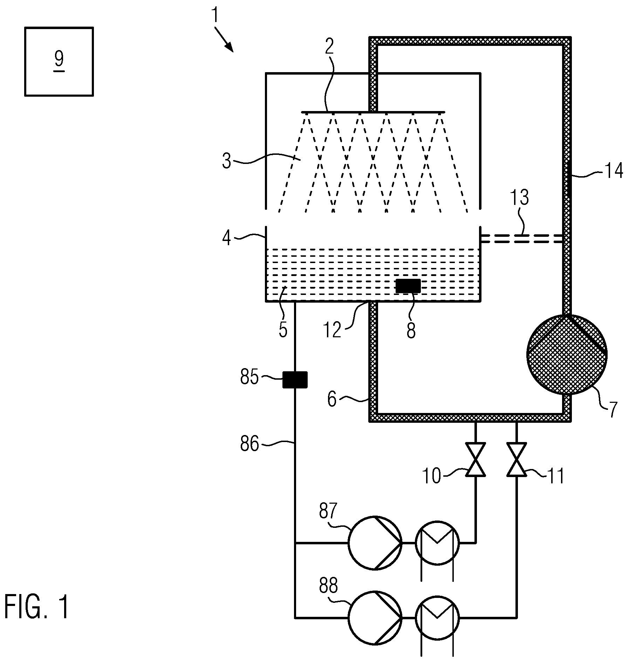

Die Figur 1 zeigt eine schematische Ansicht einer Behandlungszone 1 im Normalbetrieb. Die Behandlungszone 1 kann von einer thermischen Behälterbehandlungsvorrichtung, wie einem Kühler, einem Wärmer oder einem Pasteur, umfasst sein. Beispielsweise kann diese Behandlungszone 1 als eine Pasteurisierungszone eingesetzt werden.

Die Behandlungszone 1 umfasst eine Spritzvorrichtung 2 zum Ausbringen von Behandlungsmedium 3, beispielsweise auf Behälter, die durch die Behandlungszone 1 hindurchtransportiert werden können. Beispielsweise kann die Spritzvorrichtung 2 Düsen oder andere Ausbringmittel umfassen, mittels derer das Behandlungsmedium 3 ausgebracht werden kann.

Das durch die Spritzvorrichtung 2 ausgebrachte Behandlungsmedium 3 gelangt zumindest zum Teil in ein Becken 4, das zum Auffangen des ausgebrachten Behandlungsmediums 3 und zum Bereitstellen von Behandlungsmedium 5, unterhalb der Spritzvorrichtung 2 (beispielsweise in eine Wirkrichtung der Schwerkraft betrachtet) angeordnet ist.

Eine Leitungsverbindung 6 ist zwischen dem Becken 4 und der Spritzvorrichtung 2 vorgesehen, wobei in der Leitungsverbindung 6 eine Pumpe 7 vorgesehen ist, die betrieben wird, beispielsweise mit einer Nennleistung, damit Behandlungsmedium 5 aus dem Becken 4 und durch die Leitungsverbindung 6 zu der Spritzvorrichtung 2 verbracht und dort ausgebracht werden kann. Die Leitungsverbindung 6 kann ganz oder im Wesentlichen ganz mit Behandlungsmedium 5 gefüllt sein, wie schematisch durch die Schraffur in der Leitungsverbindung 6 angedeutet wird.

Im Becken 4 ist unterhalb eines Füllniveaus des Behandlungsmediums 5 ein Temperatursensor 8 vorgesehen. Mit ihm kann eine Temperatur des Behandlungsmediums 5 im Becken 4 gemessen werden. Alternativ kann ein Temperatursensor 85 in einer rückführenden Leitung 86 zum Messen der Temperatur des Behandlungsmediums vorgesehen sein. Ein Wert der jeweils gemessenen Temperatur kann an eine Steuerungsvorrichtung 9 übermittelt werden.

Für eine Temperierung von Behandlungsmedium ist ein erstes Temperierungsventil 10 mit einem Kühlsystem und ein zweites Temperierungsventil 11 mit einem Heizsystem verbunden, wobei das erste und das zweite Temperierungsventil 10, 11 zudem mit der Leitungsverbindung 6 verbunden sind. Es kann eine gezielte Temperierung erfolgen, beispielsweise zusätzlich zu einem Wärme- oder Kälteübergang in das ausgebrachte Behandlungsmedium 3 bei Kontakt mit den Behältern. Die Temperierungsventile 10, 11 können zwischen einem Auslass 12 des Beckens 4 und der Pumpe 7 mit der Leitungsverbindung 6 verbunden sein.

Das Kühlsystem kann ein erster Wärmetauscher 87 sein, der in der rückführenden Leitung 86 vor dem ersten Temperierungsventil 10 angeordnet ist. Das Heizsystem kann ein zweiter Wärmetauscher 88 sein, der in der rückführenden Leitung 86 vor dem zweiten Temperierungsventil 11 angeordnet ist.

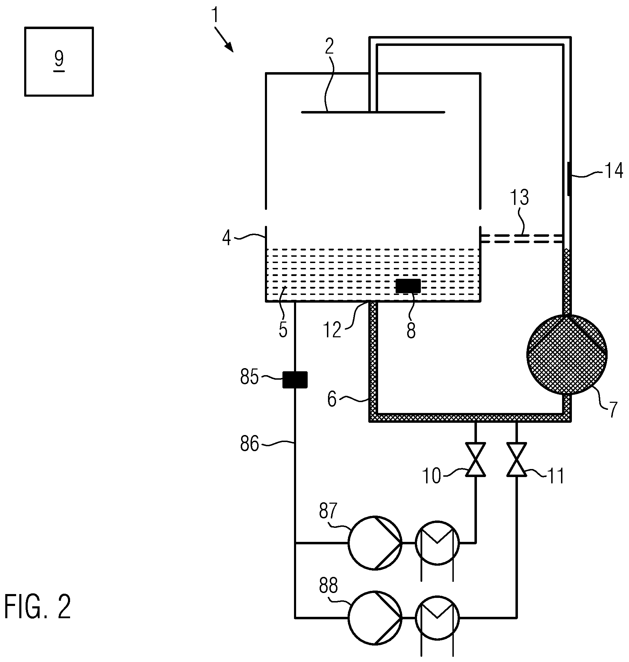

Optional kann ein Bypass 13 zwischen der Leitungsverbindung 6 und dem Becken 4 vorgesehen sein, wobei der Bypass 13 oberhalb des Füllniveaus des Behandlungsmediums 5 im Becken 4 enden kann. Hierbei kann der Bypass 13 der Pumpe 7 nachfolgend angeordnet sein. Beispielsweise kann der Bypass 13 als ein Rohr mit einem Durchmesser kleiner als 50 Millimeter vorgesehen sein. Optional kann zudem in der Leitungsverbindung 6 zwischen der Pumpe 7 und der Spritzvorrichtung 2 eine Klappe 14 vorgesehen sein. Die Funktionsweise des Bypasses 13 und der Klappe 14 werden im Zusammenhang mit der Figur 2 näher erläutert.

Die Steuerungsvorrichtung 9 ist ausgebildet, während des Normalbetriebs die Pumpe 7 zu betreiben, beispielsweise mit der Nennleistung. Weiter ist die Steuerungsvorrichtung 9 ausgebildet, durch ein Steuern der Temperierungsventile 10, 11 warmes und/oder kühles Behandlungsmedium in die Leitungsverbindung 6 einbringen zu können. Indirekt kann dadurch die Temperatur des Behandlungsmediums 5 im Becken 4 gesteuert werden.

Die Figur 2 zeigt die Behandlungszone der Figur 1 im Stand-By-Betrieb. Die Steuerungsvorrichtung 9 ist weiter ausgebildet während des Stand-By-Betriebs die Pumpe 7 nicht zu betreiben und sie entsprechend beim Übergang vom Normalbetrieb in den Stand-By-Betrieb zu stoppen. Beim Nicht-Betrieb der Pumpe 7 wird von der Spritzvorrichtung 2 kein Behandlungsmedium ausgebracht. Da die Pumpe 7 das Behandlungsmedium in der Leitungsverbindung 6 nicht mehr umpumpt, kann die Leitungsverbindung 6 nur noch zu einem Teil mit Behandlungsmedium gefüllt sein, wie schematisch durch die Schraffur in der Leitungsverbindung 6 angedeutet wird. Die Füllung der Leitungsverbindung 6 reicht vom Becken 4 bis zu einer Höhe, die dem Füllniveau des Behandlungsmediums 5 im Becken 4 entspricht.

Ein Stand-By-Betrieb kann begonnen werden, wenn sich keine Behälter mehr in der Behandlungszone 1 befinden.

Mittels des Temperatursensors 8 kann die Temperatur des Behandlungsmediums 5 im Becken 4 gemessen und so beispielsweise überwacht werden oder alternativ kann mittels des Temperatursensors 85 die Temperatur des Behandlungsmediums in der rückführenden Leitung 86 gemessen und überwacht werden. Wenn die gemessene Temperatur unter einen vorgegebenen Sollwert abfällt, kann mittels der Steuerungsvorrichtung 9 das zweite Temperierungsventil 11 angesteuert und warmes Behandlungsmedium in die Leitungsverbindung 6 eingebracht werden. Wenn die gemessene Temperatur über einen vorgegebenen Sollwert steigt, kann mittels der Steuerungsvorrichtung 9 das erste Temperierungsventil 10 angesteuert und kühles Behandlungsmedium in die Leitungsverbindung 6 eingebracht werden.

Alternativ zum Nicht-Betreiben der Pumpe 7 während des Stand-By-Betriebs kann die Steuerungsvorrichtung 9 die Pumpe 7 mit einer reduzierten Nennleistung betreiben. Dann können der zuvor als optional angegebene Bypass 13 und die zuvor als optional angegebene Klappe 14 in der Leitungsverbindung 6 genutzt werden. Die Klappe 14 ist derart vorgesehen, dass Behandlungsmedium, das mittels der mit reduzierter Leistung betriebenen Pumpe 7 aus dem Becken 4 abgepumpt werden kann, die Klappe 14 nicht passieren kann, sondern in den Bypass 13 umgelenkt werden kann. Die Höhe der reduzierten Nennleistung kann von der verwendeten Klappe 14 und/oder einem Durchmesser des Bypasses 13 abhängen.

Beim Betreiben der Pumpe 7 mit der Nennleistung kann die Klappe 14 von dem Behandlungsmedium passiert werden. Eine Umlenkung von Behandlungsmedium in den Bypass 13 kann beim Betreiben der Pumpe 7 mit der Nennleistung minimal sein oder ganz unterbunden sein.

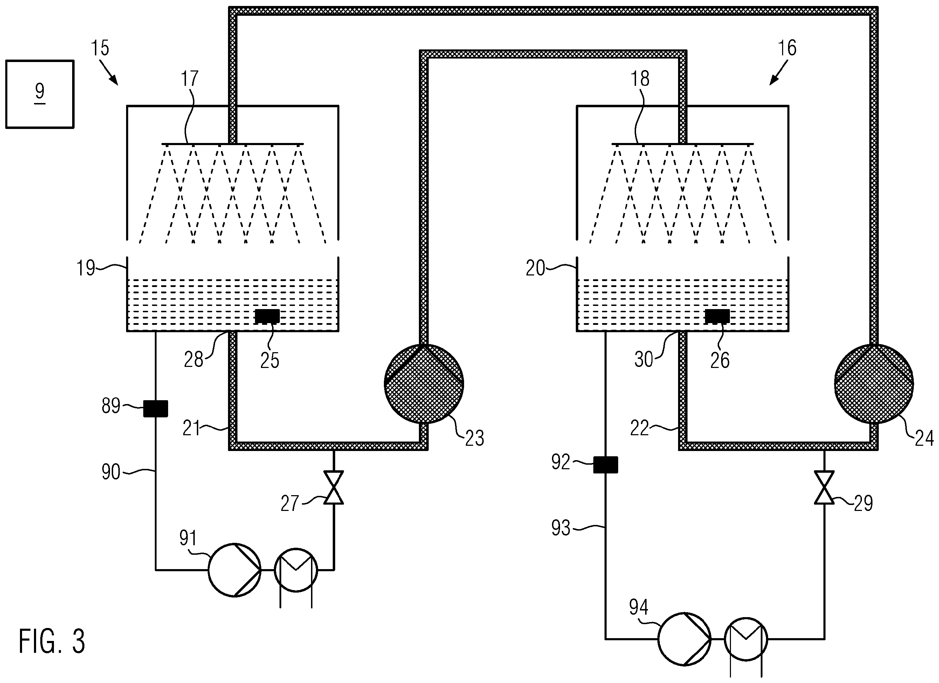

Die Figur 3 zeigt eine schematische Ansicht zweier Behandlungszonen 15, 16, die als Rekuperationsstufe geschaltet sind, im Normalbetrieb. Die erste Behandlungszone 15 kann eine Aufwärmzone, beispielsweise eine erste Aufwärmzone eines Pasteurs, sein. Die zweite Behandlungszone 16 kann eine Abkühlzone, beispielsweise eine letzte Aufwärmzone eines Pasteurs, sein.

Die erste und die zweite Behandlungszone 15, 16 umfassen jeweils eine Spritzvorrichtung 17, 18 zum Ausbringen von Behandlungsmedium. Beispielsweise können die Spritzvorrichtungen 17, 18 Düsen oder andere Ausbringmittel umfassen, mittels derer das Behandlungsmedium ausgebracht werden kann.

Jeweils bei der ersten und der zweiten Behandlungszone 15, 16 gelangt das durch die jeweilige Spritzvorrichtung 17, 18 ausgebrachte Behandlungsmedium zumindest zum Teil in ein Becken 19, 20 dieser Behandlungszone, das zum Auffangen des ausgebrachten Behandlungsmediums und zum Bereitstellen von Behandlungsmedium, unterhalb der jeweiligen Spritzvorrichtung 17, 18 (beispielsweise in eine Wirkrichtung der Schwerkraft betrachtet) dieser Behandlungsvorrichtung 15, 16 angeordnet ist.

Das Becken 19 der ersten Behandlungszone 15 ist mit der Spritzvorrichtung 18 der zweiten Behandlungszone 16 mittels einer ersten Leitungsverbindung 21 verbunden und das Becken 20 der der zweiten Behandlungszone 16 ist mit der Spritzvorrichtung 17 der ersten Behandlungszone 15 mittels einer zweiten Leitungsverbindung 22 verbunden.

In der ersten Leitungsverbindung 21 ist eine erste Pumpe 23 vorgesehen, die betrieben wird, beispielsweise mit einer Nennleistung durch die Steuerungsvorrichtung 9, damit Behandlungsmedium aus dem Becken 19 der ersten Behandlungszone 15 und durch die erste Leitungsverbindung 21 zu der Spritzvorrichtung 18 der zweiten Behandlungszone 16 verbracht und dort ausgebracht werden kann. Die erste Leitungsverbindung 21 kann ganz oder im Wesentlichen ganz mit Behandlungsmedium gefüllt sein.

In der zweiten Leitungsverbindung 22 ist eine zweite Pumpe 24 vorgesehen, die betrieben wird, beispielsweise mit einer Nennleistung durch die Steuerungsvorrichtung 9, damit Behandlungsmedium aus dem Becken 20 der zweiten Behandlungszone 16 und durch die zweite Leitungsverbindung 22 zu der Spritzvorrichtung 17 der ersten Behandlungszone 15 verbracht und dort ausgebracht werden kann. Die zweite Leitungsverbindung 22 kann ganz oder im Wesentlichen ganz mit Behandlungsmedium gefüllt sein.

In den Becken 19, 20 der ersten und zweiten Behandlungszone 15, 16 ist jeweils unterhalb eines Füllniveaus des Behandlungsmediums ein Temperatursensor 25, 26 vorgesehen. Mit ihm kann eine Temperatur des Behandlungsmediums im entsprechenden Becken 19, 20 gemessen werden. Alternativ kann jeweils ein Temperatursensor 89, 92 in den rückführenden Leitungen 90, 93 zum Messen der Temperatur des Behandlungsmediums vorgesehen sein. Ein jeweiliger Wert der jeweiligen gemessenen Temperatur kann an die Steuerungsvorrichtung 9 übermittelt werden.

Für eine Temperierung des Behandlungsmediums im Becken 19 der ersten Behandlungszone 15 ist ein erstes Temperierungsventil 27 mit einem Kühlsystem und der ersten Leitungsverbindung 21 verbunden. Es kann eine gezielte Temperierung erfolgen, beispielsweise zusätzlich zu einem Wärme- oder Kälteübergang in das ausgebrachte Behandlungsmedium bei Kontakt mit den Behältern. Das erste Temperierungsventil 27 kann zwischen einem Auslass 28 aus dem Becken 19 der ersten Behandlungszone 15 und der ersten Pumpe 23 mit der ersten Leitungsverbindung 21 verbunden sein.

Für eine Temperierung des Behandlungsmediums im Becken 20 der zweiten Behandlungszone 16 ist ein zweites Temperierungsventil 29 mit einem Heizsystem und der zweiten Leitungsverbindung 22 verbunden. Es kann eine gezielte Temperierung erfolgen, beispielsweise zusätzlich zu einem Wärme- oder Kälteübergang in das ausgebrachte Behandlungsmedium bei Kontakt mit den Behältern. Das zweite Temperierungsventil 29 kann zwischen einem Auslass 30 aus dem Becken 20 der zweiten Behandlungszone 16 und der zweiten Pumpe 24 mit der zweiten Leitungsverbindung 22 verbunden sein.

Das Kühlsystem kann ein erster Wärmetauscher 91 sein, der in der rückführenden Leitung 90 vor dem ersten Temperierungsventil 27 angeordnet ist. Das Heizsystem kann ein zweiter Wärmetauscher 94 sein, der in der rückführenden Leitung 93 vor dem zweiten Temperierungsventil 29 angeordnet ist.

Die Steuerungsvorrichtung 9 ist ausgebildet, während des Normalbetriebs die Pumpen 23, 24 zu betreiben, beispielsweise mit der Nennleistung, sodass durch die Spritzvorrichtungen 17, 18 der ersten und zweiten Behandlungszonen 15, 16 jeweils Behandlungsmedium ausgebracht wird. Weiter ist die Steuerungsvorrichtung 9 ausgebildet, durch ein Steuern des ersten Temperierungsventils 27 der ersten Behandlungszone 15 kühles Behandlungsmedium in die erste Leitungsverbindung 21 und durch ein Steuern des zweiten Temperierungsventils 29 der zweiten Behandlungszone 16 warmes Behandlungsmedium in die zweite Leitungsverbindung 22 einbringen zu können. Indirekt kann dadurch jeweils die Temperatur des Behandlungsmediums im Becken 19, 20 der ersten und der zweiten Behandlungszone 15, 16 gesteuert werden.

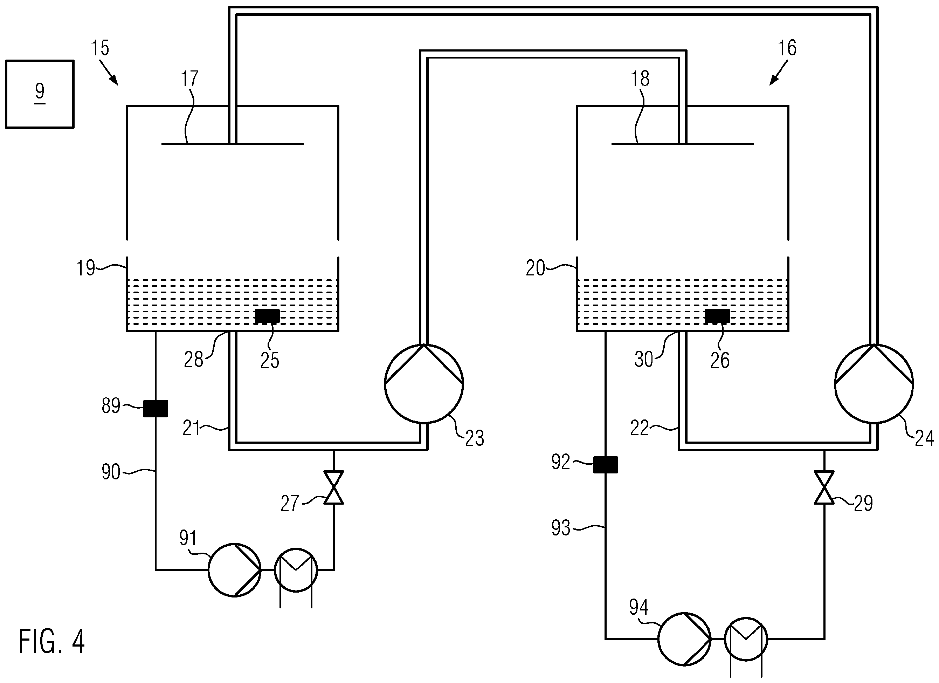

Die Figur 4 zeigt die zwei Behandlungszonen 15, 16 der Figur 3 im Stand-By-Betrieb. Die erste und die zweite Pumpe 23, 24 werden durch die Steuerungsvorrichtung 9 nicht betrieben, sodass durch die Spritzvorrichtungen 17, 18 der ersten und der zweiten Behandlungszone 15, 16 jeweils kein Behandlungsmedium ausgebracht wird. Das erste Temperierungsventil 27 der ersten Behandlungszone 15 und das zweite Temperierungsventil 29 der zweiten Behandlungszone 16 werden weiterhin mittels der Steuerungsvorrichtung 9 geregelt.

Mittels des Temperatursensors 25 im Becken 19 der ersten Behandlungszone 15 kann die Temperatur des Behandlungsmediums in diesem Becken 19 gemessen und so beispielsweise überwacht werden oder alternativ kann mittels des Temperatursensors 89 die Temperatur des Behandlungsmediums in der rückführenden Leitung 90 gemessen und überwacht werden. Wenn die gemessene Temperatur über einen vorgegebenen Sollwert steigt, kann mittels der Steuerungsvorrichtung 9 das erste Temperierungsventil 27 der ersten Behandlungszone 15 angesteuert und kühles Behandlungsmedium in die erste Leitungsverbindung 21 eingebracht werden.

Mittels des Temperatursensors 26 im Becken 20 der zweiten Behandlungszone 16 kann die Temperatur des Behandlungsmediums in diesem Becken 20 gemessen und so beispielsweise überwacht werden oder alternativ kann mittels des Temperatursensors 92 die Temperatur des Behandlungsmediums in der rückführenden Leitung 93 gemessen und überwacht werden. Wenn die gemessene Temperatur unter einen vorgegebenen Sollwert fällt, kann mittels der Steuerungsvorrichtung 9 das zweite Temperierungsventil 29 der zweiten Behandlungszone 16 angesteuert und warmes Behandlungsmedium in die zweite Leitungsverbindung 22 eingebracht werden.

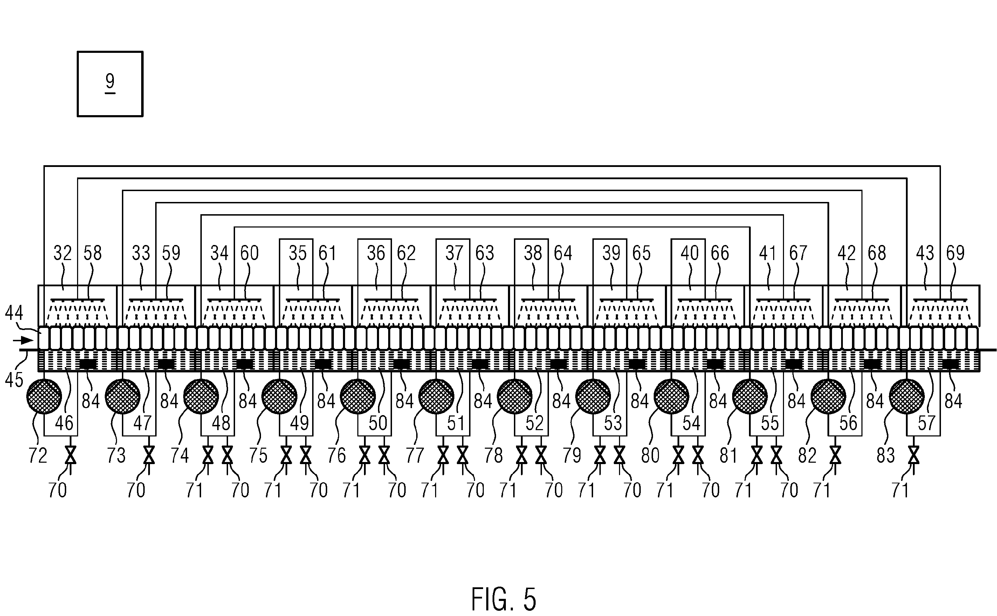

Die Figur 5 zeigt eine schematische Ansicht eines Pasteurs 31 (als thermische Behälterbehandlungsvorrichtung) mit drei Aufwärmzonen- 32, 33, 34, sechs Pasteurisierungszonen 35, 36, 37, 38, 39, 40 und drei Abkühlzonen 41, 42, 43 im Normalbetrieb. Behälter 44 werden, beispielsweise auf einem Transportband 45, durch den Pasteur 31 geführt und in den verschiedenen Zonen 32-43 mit Behandlungsmedium besprüht. Die Behälter 44 können aufeinanderfolgend die erste, zweite, dritte Aufwärmzone 32-34, die erste, zweite, dritte, vierte, fünfte und sechste Pasteurisierungszone 35-40, die erste, zweite und dritte Abkühlzone 41-43 durchlaufen. Das hier und im Folgenden Beschriebene kann entsprechend auch für einen Pasteur mit weniger oder mehr Aufwärm- und Abkühlzonen und mit weniger oder mehr Pasteurisierungszonen gelten.

Die erste Aufwärmzone 32 und die dritte Abkühlzone 43, die zweite Aufwärmzone 33 und die zweite Abkühlzone 42 sowie die dritte Aufwärmzone 34 und die erste Abkühlzone 43 werden jeweils als Rekuperationsstufe betrieben. Das Becken 46 der ersten Aufwärmzone 32 ist mit der Spritzvorrichtung 69 der dritten Abkühlzone 43 und das Becken 57 der der dritten Abkühlzone 43 ist mit der Spritzvorrichtung 58 der ersten Aufwärmzone 32 leitungsverbunden. Das Becken 47 der zweiten Aufwärmzone 33 ist mit der Spritzvorrichtung 68 der zweiten Abkühlzone 42 und das Becken 56 der der zweiten Abkühlzone 42 ist mit der Spritzvorrichtung 59 der zweiten Aufwärmzone 33 leitungsverbunden Das Becken 48 der dritten Aufwärmzone 34 ist mit der Spritzvorrichtung 67 der ersten Abkühlzone 41 und das Becken 55 der der ersten Abkühlzone 41 ist mit der Spritzvorrichtung 60 der dritten Aufwärmzone 34 leitungsverbunden.

Bei den sechs Pasteurisierungszonen 35- 40 ist ein Becken 49-54 einer der Pasteurisierungszonen 35-40 mit der Spritzvorrichtung 61-66 der gleichen Pasteurisierungszone 35-40 leitungsverbunden.

In den Becken 46-57 ist jeweils ein Temperatursensor 84 angeordnet mit dem jeweils die Temperatur des im Becken 46-57 vorhandenen Behandlungsmediums gemessen und überwacht werden kann. Alternativ kann anstatt der Temperatursensoren 84 in den Becken jeweils ein Temperatursensor (nicht dargestellt) in der rückführenden Leitung vorgesehen sein.

Die erste und zweite Aufwärmzone 32, 33 umfassen jeweils ein zweites Temperierungsventil 70, das mit einem Heizsystem verbunden ist.

Die dritte Aufwärmzone 34, die sechs Pasteurisierungszonen 35-40 und die erste Abkühlzone 41 umfassen jeweils ein erstes Temperierungsventil 71, das mit einem Kühlsystem verbunden ist, und ein zweites Temperierungsventil 70, das mit einem Heizsystem verbunden ist.

Die zweite und dritte Abkühlzone 42, 43 umfassen jeweils ein erstes Temperierungsventil 71, das mit einem Kühlsystem verbunden ist.

Während des Normalbetriebs wird die jeweilige Pumpe 72-83 von allen Behandlungszonen 32-43 betrieben und die ersten und zweiten Temperierungsventile 71, 70 werden jeweils gesteuert.

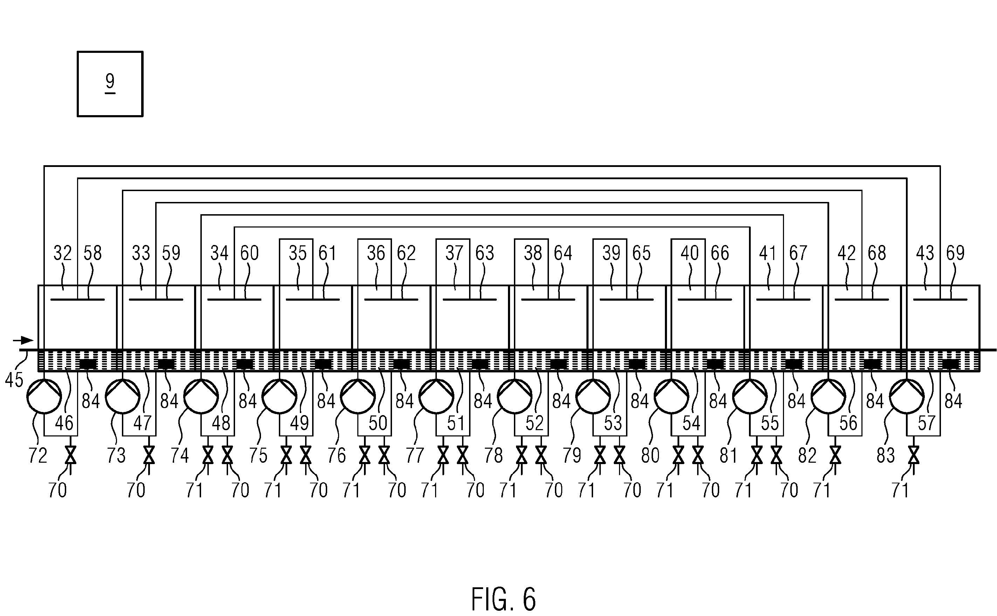

Die Figur 6 zeigt den Pasteur der Figur 5 im Stand-By-Betrieb. Hierbei sind alle Behandlungszonen 32-43 im Stand-By-Betrieb, weshalb auch gesagt werden kann, dass der Pasteur 31 im Stand-By-Betrieb ist. Im Stand-By-Betrieb werden alle Pumpen 72-83 nicht betrieben, die ersten und zweiten Temperierungsventile 71, 70 werden jedoch weiterhin gesteuert, um die Temperatur des Behandlungsmediums in den jeweiligen Becken 46-57 gegebenenfalls auf einem Sollwert oder in einem Sollwertebereich der Temperatur halten zu können, wenn durch die Spritzvorrichtungen 58-69 kein Behandlungsmedium mehr ausgegeben wird, das in die jeweiligen Becken 46-57 gelangen kann. Der Stand-By-Betrieb aller Behandlungszonen 32-43 des Pasteurs 31 kann vorgesehen sein, wenn sich keine Behälter zum Behandeln im Pasteur 31 befinden. Der in der Figur 5 dargestellte Normalbetrieb des Pasteurs 31 kann mittels der Steuerungsvorrichtung 9, nachdem der letzte Behälter den Pasteur 31 verlassen hat, durch Stoppen der Pumpen 72-83 und dann Nicht-Betreiben der Pumpen 72-83 beendet werden, um in den Stand-By-Betrieb überzugehen.

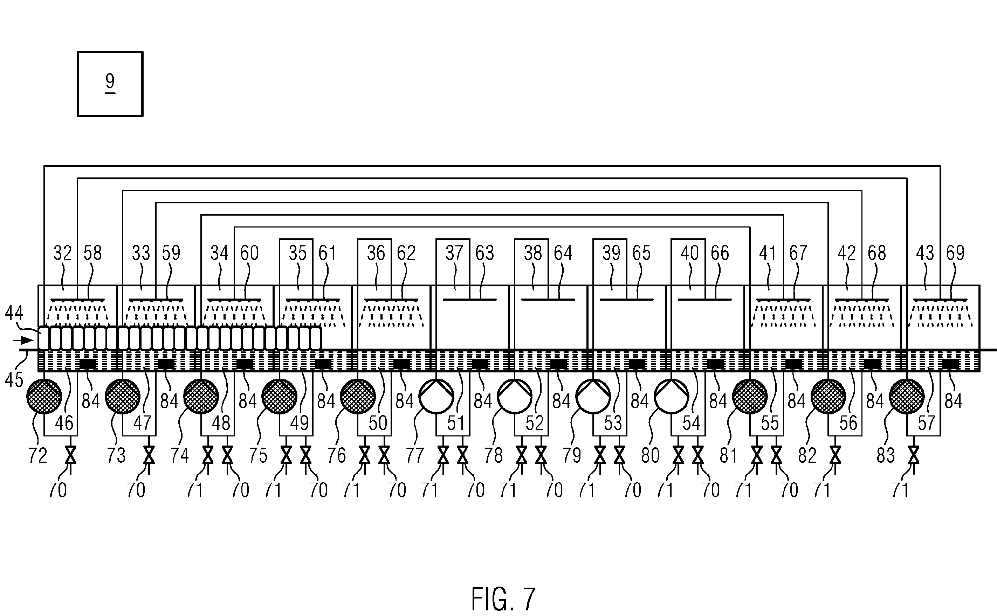

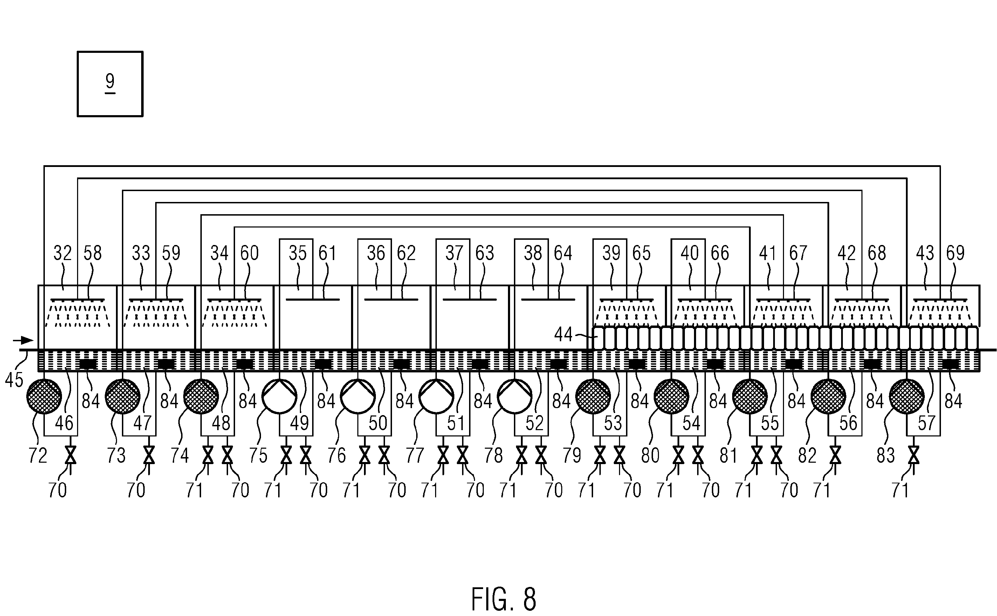

Im Zusammenhang mit den Figuren 7 und 8 werden Aspekte erläutert, wie einzelne Pasteurisierungszonen 35-40 und/oder Rekuperationsstufen aus dem Stand-By-Betrieb in den Normalbetrieb oder aus dem Normalbetrieb in den Stand-By-Betrieb gebracht werden können, beispielsweise unter Berücksichtigung eines Zeitpunkts, zu dem Behälter 44 in den Pasteur 31 eingebracht werden und/oder unter Berücksichtigung eines Zeitpunkts, wann Behälter 44 bei einer bestimmten Behandlungszone 32-43 eintreffen werden.

Die Figur 7 zeigt den Pasteur 31 der Figur 5 beim Einlaufen von Behältern 44, wobei sich zu dem dargestellten Zeitpunkt bereits Behälter 44 in den ersten bis dritten Aufwärmzonen 32-34 und einige Behälter 44 in der ersten Pasteurisierungszone 35 befinden. Das dargestellte Einlaufen der Behälter 44 kann anschließend an einen Stand-By-Betrieb des Pasteurs 31, wie in der Figur 6 dargestellt, erfolgen.

Beispielsweise wurden mittels der Steuerungsvorrichtung die Pumpe 72 der ersten Aufwärmzone 32 und die Pumpe 83 der dritten Abkühlzone 43 zu einem ersten Zeitpunkt in Betrieb genommen, der einer geplanten Zufuhr von Behältern 44 (der Zufuhrzeitpunkt kann bekannt sein), nach einem Stand-By-Betrieb des Pasteurs 31, vorausgeht. Die Zeitdauer, die zwischen einer Inbetriebnahme der Pumpen 72, 83 und dem Eintreffen des ersten Behälters 44 in der ersten Aufwärmzone 32 verstreicht, kann als Vorlaufdauer für ein Sicheinstellen Regelung der Rekuperationsstufe aus erster Aufwärmzone 32 und dritter Abkühlzone 43 angesehen werden. So kann es möglich sein, dass sich die Regelung der Rekuperationsstufe aus erster Aufwärmzone 32 und dritter Abkühlzone 43 während dieser Vorlaufdauer auf die bevorstehende Behandlung der Behälter 44 mit Behandlungsmedium für den Normalbetrieb einstellen kann. Durch den Betrieb der Pumpe 72 der ersten Aufwärmzone 32 und der Pumpe 83 der dritten Abkühlzone 43 wird durch die Spritzvorrichtung 58 der ersten Aufwärmzone 32 und die Spritzvorrichtung 69 der dritten Abkühlzone 43 Behandlungsmedium ausgebracht, das in der ersten Aufwärmzone 32 auf die dort vorhandenen Behälter 44 aufgebracht werden kann.

Solch eine Vorlaufdauer kann auch für die weiteren Aufwärmzonen 33, 34 und die Pasteurisierungszonen 35-40 vorgesehen sein. Für unterschiedliche Zonen können gleichlange oder verschieden lange Vorlaufdauern vorgesehen sein.

Die der ersten Aufwärmzone 32 nachfolgende zweite Aufwärmzone 33 wurde zusammen mit der zweiten Abkühlzone 42 zeitverzögert zu dem ersten Zeitpunkt, beispielsweise zu einem zweiten Zeitpunkt, in den Normalbetrieb genommen, indem mittels der Steuerungsvorrichtung 9 die Pumpe 72 der zweiten Aufwärmzone 33 und die Pumpe 82 der zweiten Abkühlzone 42 zum zweiten Zeitpunkt in Betrieb genommen wurden. Der zweite Zeitpunkt kann beispielsweise unter Beachtung einer bekannten oder abgeschätzten Transportdauer der Behälter 44 durch die erste Aufwärmzone 33 hindurch, d.h. bis ein erster Behälter 44 aus der ersten Aufwärmzone 32 bei der zweiten Aufwärmzone 33 eintreffen kann, und unter Beachtung einer gewünschten Vorlaufdauer ermittelt werden. Durch den Betrieb der Pumpe 73 der zweiten Aufwärmzone 33 und der Pumpe 82 der zweiten Abkühlzone 42 wird durch die Spritzvorrichtung 59 der zweiten Aufwärmzone 33 und der Spritzvorrichtung 68 der zweiten Abkühlzone 42 Behandlungsmedium ausgebracht, das in der zweiten Aufwärmzone 33 auf die Behälter 44 aufgebracht werden kann.

Die der zweiten Aufwärmzone 33 nachfolgende dritte Aufwärmzone 34 wurde zusammen mit der ersten Abkühlzone 41 zeitverzögert zu dem zweiten Zeitpunkt, beispielsweise zu einem dritten Zeitpunkt, in den Normalbetrieb genommen, indem mittels der Steuerungsvorrichtung 9 die Pumpe 74 der dritten Aufwärmzone 34 und die Pumpe 81 der ersten Abkühlzone 41 zum dritten Zeitpunkt in Betrieb genommen wurden. Der dritte Zeitpunkt kann beispielsweise unter Beachtung einer bekannten oder abgeschätzten Transportdauer der Behälter 44 durch die zweite Aufwärmzone 33, d.h. bis ein erster Behälter 44 aus der zweiten Aufwärmzone 33 bei der dritten Aufwärmzone 34 eintreffen kann, und unter Beachtung einer gewünschten Vorlaufdauer ermittelt werden. Durch den Betrieb der Pumpe 74der dritten Aufwärmzone 34 und der Pumpe 81 der ersten Abkühlzone 41 wird durch die Spritzvorrichtung 60 der dritten Aufwärmzone 34 und die Spritzvorrichtung 67 der ersten Abkühlzone 41 Behandlungsmedium ausgebracht, das in der dritten Aufwärmzone 34 auf die Behälter 44 aufgebracht werden kann.

Die der dritten Aufwärmzone 34 nachfolgende erste Pasteurisierungszone 35 wurde zeitverzögert zu dem dritten Zeitpunkt, beispielsweise zu einem vierten Zeitpunkt, in den Normalbetrieb genommen, indem mittels der Steuerungsvorrichtung 9 die Pumpe 75 der ersten Pasteurisierungszone 35 zum vierten Zeitpunkt in Betrieb genommen wurde. Der vierte Zeitpunkt kann beispielsweise unter Beachtung einer bekannten oder abgeschätzten Transportdauer der Behälter 44 durch die dritte Aufwärmzone 34, d.h. bis ein erster Behälter 44 aus der dritten Aufwärmzone 34 bei der ersten Pasteurisierungszone 35 eintreffen kann, und unter Beachtung einer gewünschten Vorlaufdauer ermittelt werden. Durch den Betrieb der Pumpe 75 der ersten Pasteurisierungszone 35 wird durch die Spritzvorrichtung 61 der ersten Pasteurisierungszone 35 Behandlungsmedium ausgebracht und auf die Behälter 44 aufgebracht.

Die Zeitdauer, die zwischen der Inbetriebnahme der Pumpe 75 der ersten Pasteurisierungszone 35 und dem Eintreffen des ersten Behälters 44 in der ersten Pasteurisierungszone 35 verstreicht, kann hier als Vorlaufdauer für ein Sicheinstellen Regelung der ersten Pasteurisierungszone 35 angesehen werden. So kann es möglich sein, dass sich die Regelung der ersten Pasteurisierungszone 35 während dieser Vorlaufdauer auf die bevorstehende Behandlung der Behälter 44 mit Behandlungsmedium für den Normalbetrieb einstellen kann.

In der Darstellung der Figur 7 befinden sich Behälter 44 in der ersten, zweiten und dritten Aufwärmzone 32-34 und die erste Pasteurisierungszone 35 haben Behälter 44 erst zum Teil passiert.

Die zweite Pasteurisierungszone 36 wurde ebenfalls schon in den Normalbetrieb versetzt, indem durch die Steuerungsvorrichtung 9 die Pumpe 76 der zweiten Pasteurisierungszone 36 in Betrieb genommen wurde, sodass Behandlungsmedium mittels der Spritzvorrichtung 62 der zweiten Pasteurisierungszone 36 ausgebracht werden kann. Die der ersten Pasteurisierungszone 35 nachfolgende zweite Pasteurisierungszone 36 wurde zeitverzögert zu dem vierten Zeitpunkt, beispielsweise zu einem fünften Zeitpunkt, in den Normalbetrieb genommen, indem mittels der Steuerungsvorrichtung 9 die Pumpe 76 wie beschrieben in Betrieb genommen wurde. Der fünfte Zeitpunkt kann beispielsweise unter Beachtung einer bekannten oder abgeschätzten Transportdauer der Behälter 44 durch die erste Pasteurisierungszone 35, d.h. bis ein erster Behälter 44 aus der ersten Pasteurisierungszone 35 bei der zweiten Pasteurisierungszone 36 eintreffen kann, und unter Beachtung einer gewünschten Vorlaufdauer ermittelt werden.

Für das Inbetriebnehmen können die Pumpen 72-76, 81-83 jeweils mit einer Nennleistung betrieben werden.

Die dritte, vierte, fünfte und sechste Pasteurisierungszone 37-40 sind jeweils noch im Stand-By-Betrieb und können gemäß der Beschreibung für die erste und zweite Pasteurisierungszone 35, 36 ebenso in den Normalbetrieb genommen werden.

Die Figur 8 zeigt den Pasteur 31 der Figur 5 beim Auslaufen von Behältern 44. Behälter 44 sind in der Darstellung nur noch in der fünften und sechsten Pasteurisierungszone 39, 40 und in den drei Abkühlzonen 41-43 vorhanden.

Die erste, zweite, dritte und vierte Pasteurisierungszone 35-38 sind bereits im Stand-By-Betrieb. Durch die Steuerungsvorrichtung 9 wurden die Pumpen 75-78 dieser Pasteurisierungszonen 35-38 nacheinander nicht mehr betrieben, sobald die Behälter 44 eine entsprechende Pasteurisierungszone 35-38 verlassen hatten, sodass entsprechend kein Behandlungsmedium mehr durch die entsprechend Spritzvorrichtung 61-64 ausgebracht wurde.

Die erste, zweite und dritte Aufwärmzone 32-34 befinden sich weiterhin im Normalbetrieb, da sie zusammen mit der dritten, zweiten und ersten Abkühlzone 43, 42, 41 jeweils eine Rekuperationsstufe bilden und sich in der ersten, zweiten und dritten Abkühlzone 41-43 noch Behälter 44 befinden.

Wenn die Behälter 44 die fünfte Pasteurisierungszone 39 verlassen haben, kann diese in den Stand-By-Betrieb versetzt werden und wenn die Behälter 44 die sechste Pasteurisierungszone 40 verlassen haben, kann diese dann in den Stand-By-Betrieb versetzt werden.

Wenn die Behälter 44 die erste Abkühlzone 41 verlassen haben, können die erste Abkühlzone 41 und die dritte Aufwärmzone 34 in den Stand-By-Betrieb versetzt werden.

Wenn die Behälter 44 die zweite Abkühlzone 42 verlassen haben, können die zweite Abkühlzone 42 und die zweite Aufwärmzone 33 in den Stand-By-Betrieb versetzt werden.

Wenn die Behälter 44 die dritte Abkühlzone 43 verlassen haben, können die dritte Abkühlzone 43 und die erste Aufwärmzone 32 in den Stand-By-Betrieb versetzt werden.

Der Pasteur 31 befindet sich dann wieder im Stand-By-Betrieb, wie er beispielsweise in der Figur 6 dargestellt wurde.

## Patentansprüche

1. Thermische Behälterbehandlungsvorrichtung, wie Kühler, Wärmer oder Pasteur, umfassend:
- eine Fördervorrichtung (45) für Behälter (44),
- eine Behandlungszone (1, 15, 17, 32-43) mit einer Spritzvorrichtung (2, 17, 18, 58-69) zum Ausbringen von Behandlungsmedium auf Behälter (44),
- ein Becken (4, 19, 20, 46-57) zum Auffangen und Bereitstellen von Behandlungsmedium,
- eine Leitungsverbindung (6, 21, 22) zwischen Becken (4, 19, 20) und Spritzvorrichtung (2, 17, 18), wobei in der Leitungsverbindung eine Pumpe (7, 23, 24) vorgesehen ist,
**dadurch gekennzeichnet, dass**
- ein Temperatursensor (8, 25, 26, 84, 85, 89, 92) zur Messung einer Temperatur des Behandlungsmediums vorgesehen ist,
- die thermische Behälterbehandlungsvorrichtung weiter eine Steuerungsvorrichtung (9) umfasst, die ausgebildet ist,
während eines Normalbetriebs in der Behandlungszone die Pumpe mit einer Nennleistung zum Ausbringen von Behandlungsmedium mittels der Spritzvorrichtung zu betreiben und
während eines Stand-By-Betriebs in der Behandlungszone die Pumpe nicht zu betreiben oder die Pumpe mit einer reduzierten Nennleistung zu betreiben, ohne dass Behandlungsmedium mittels der Spritzvorrichtung ausbringbar ist.

2. Die thermische Behälterbehandlungsvorrichtung nach Anspruch 1, wobei die Leitungsverbindung im Becken mit einem Saugstutzen endet und wobei der Temperatursensor (8, 25, 26, 84) benachbart zu dem Saugstutzen angeordnet ist und/oder
wobei ein Bypass (13) zwischen der Leitungsverbindung (6) und dem Becken (4) vorgesehen ist, wobei der Bypass (13) oberhalb des Füllniveaus des Behandlungsmediums im Becken (4) endet, wobei beispielsweise der Bypass (13) der Pumpe (7) nachfolgend angeordnet ist, wobei beispielsweise in der Leitungsverbindung (6) zwischen der Pumpe (7) und der Spritzvorrichtung (2) eine Klappe 14) vorgesehen ist, wobei beispielsweise der Bypass (13) als ein Rohr mit einem Durchmesser kleiner als 50 Millimeter vorgesehen ist.

3. Die thermische Behälterbehandlungsvorrichtung nach Anspruch 1 oder 2, wobei die Leitungsverbindung zudem eine Verbindung zu mindestens einem Temperierungsventil (10, 11, 27, 29, 70, 71) aufweist, wobei das mindestens eine Temperierungsventil mit einem Heiz- und/oder Kühlsystem verbunden ist für eine Bereitstellung von temperiertem Behandlungsmedium.

4. Die thermische Behälterbehandlungsvorrichtung nach Anspruch 3, wobei die Steuerungsvorrichtung (9) weiter ausgebildet ist, durch ein Steuern des mindestens einen Temperierungsventils (10, 11, 27, 29, 70, 71) eine Temperatur des Behandlungsmediums im Becken zu steuern.

5. Die thermische Behälterbehandlungsvorrichtung nach einem der Ansprüche 1 bis 4, umfassend mehrere der Behandlungszonen, wobei die Behandlungszonen als Aufwärmzonen oder als Abkühlzonen ausgebildet sind.

6. Die thermische Behälterbehandlungsvorrichtung nach einem der Ansprüche 1 bis 4, umfassend mehrere der Behandlungszonen (32-43),
wobei mindestens eine davon als eine Aufwärmzone (32-34), mindestens eine davon als eine Pasteurisierungszone (35-40) und mindestens eine davon als eine Abkühlzone (41-43) ausgebildet ist,
wobei die mindestens eine Aufwärmzone (32-34) und die mindestens eine Abkühlzone (41-43) zusammen als eine Rekuperationsstufe ausgebildet sind,
wobei eine erste Leitungsverbindung von einem Becken einer Aufwärmzone zu einer Spritzvorrichtung einer Abkühlzone, wobei eine zweite Leitungsverbindung von einem Becken einer Abkühlzone zu einer Spritzvorrichtung einer Aufwärmzone und eine dritte Leitungsverbindung von einem Becken einer Pasteurisierungszone zur Spritzvorrichtung dieser Pasteurisierungszone vorgesehen ist.

7. Die thermische Behälterbehandlungsvorrichtung nach Anspruch 5 oder 6, wobei die Steuerungsvorrichtung (9) weiter ausgebildet ist, während eines Stand-By-Betriebs der thermischen Behälterbehandlungsvorrichtung alle Pumpen (72-83) nicht zu betreiben oder alle Pumpen (72-83) mit einer reduzierten Nennleistung zu betreiben zum Nicht-Ausbringen von Behandlungsmedium mittels der jeweiligen Spritzvorrichtung.

8. Die thermische Behälterbehandlungsvorrichtung nach Anspruch 7, soweit rückbezogen auf Anspruch 6, wobei die Steuerungsvorrichtung (9) weiter ausgebildet ist nach einem Stand-by-Betrieb der thermischen Behälterbehandlungsvorrichtung zum Erreichen eines Normalbetriebs:
- zu einem ersten gegebenen Zeitpunkt vor einem geplanten Zuführen von Behältern (44) zu der mindestens einen Aufwärmzone, die Pumpen der mindestens einen Aufwärmzone und der mindestens einen Abkühlzone mit der Nennleistung zu betreiben zum Ausbringen von Behandlungsmedium aus den Spritzvorrichtungen der mindestens einen Aufwärmzone und der mindestens einen Abkühlzone, und danach
- zu einem zweiten gegebenen Zeitpunkt vor einem geplanten Zuführen von Behältern (44) aus der mindestens einen Anwärmzone zu der mindestens einen Pasteurisierungszone, die Pumpe der mindestens einen Pasteurisierungszone mit der Nennleistung zu betreiben zum Ausbringen von Behandlungsmedium aus der Spritzvorrichtung der mindestens einen Pasteurisierungszone.

9. Die thermische Behälterbehandlungsvorrichtung nach Anspruch 8, wobei die Steuerungsvorrichtung (9) nach Erreichen des Normalbetriebs der thermischen Behandlungsanlage weiter ausgebildet ist zum Erreichen eines Stand-By-Betriebs:
- nachdem Behälter (44) die mindestens eine Pasteurisierungszone verlassen haben, die Pumpe der mindestens einen Pasteurisierungszone nicht zu betreiben oder die Pumpe der mindestens einen Pasteurisierungszone mit einer reduzierten Nennleistung zu betreiben zum Nicht-Ausbringen von Behandlungsmedium mittels der Spritzvorrichtung der mindestens einen Pasteurisierungszone, und danach
- nachdem Behälter (44) die letzte der mindestens einen Abkühlzone verlassen haben, alle Pumpen der mindestens einen Aufwärmzone und der mindestens einen Abkühlzone nicht zu betreiben oder alle Pumpen der mindestens einen Aufwärmzone und der mindestens einen Abkühlzone mit einer reduzierten Nennleistung zu betreiben zum Nicht-Ausbringen von Behandlungsmedium mittels der jeweiligen Spritzvorrichtungen.

10. Die thermische Behälterbehandlungsvorrichtung nach einem der Ansprüche 1 bis 9, wobei der Temperatursensor (8, 25, 26, 84) im Becken unterhalb eines Füllniveaus des Behandlungsmediums vorgesehen ist oder wobei der Temperatursensor (85, 89, 92) in einer rückführenden Leitung (86, 90, 93) vorgesehen ist.

11. Verfahren zum Regeln einer thermischen Behälterbehandlungsvorrichtung, wie Kühler, Wärmers oder Pasteur, nach einem der Ansprüche 1 bis 10.

12. Das Verfahren nach Anspruch 11, umfassend:
- während eines Stand-By-Betriebs in der Behandlungszone (1, 15, 16, 32-43), Nicht-Betreiben der Pumpe (7, 23, 24, 72-83) oder Betreiben der mit einer reduzierten Nennleistung, ohne dass Behandlungsmedium mittels der Spritzvorrichtung ausgebracht wird und/oder
- während eines Normalbetriebs in der Behandlungszone (1, 15, 16, 32-43), Betreiben der Pumpe mit einer Nennleistung zum Ausbringen von Behandlungsmedium mittels der Spritzvorrichtung.

13. Das Verfahren nach Anspruch 11 oder 12, für eine thermische Behälterbehandlungsvorrichtung nach einem der Ansprüche 3 bis 10, wobei das Verfahren ein Steuern des mindestens einen Temperierungsventils zum Steuern einer Temperatur des Behandlungsmediums im Becken umfasst.

14. Das Verfahren nach einem der Ansprüche 11 bis 13, für eine thermische Behälterbehandlungsvorrichtung nach einem der Ansprüche 5 bis 10, wobei das Verfahren umfasst:
- während eines Stand-By-Betriebs der thermischen Behälterbehandlungsvorrichtung, Nicht-Betreiben aller Pumpen oder Betreiben aller Pumpen mit einer reduzierten Nennleistung zum Nicht-Ausbringen von Behandlungsmedium mittels der jeweiligen Spritzvorrichtung.

15. Das Verfahren nach Anspruch 14, weiter umfassend, zum Erreichen eines Normalbetriebs der thermischen Behälterbehandlungsvorrichtung nach einem Stand-by-Betrieb der thermischen Behälterbehandlungsvorrichtung:
- zu einem ersten gegebenen Zeitpunkt vor einem geplanten Zuführen von Behältern (44) zu der mindestens einen Aufwärmzone, Betreiben der Pumpen der mindestens einen Aufwärmzone und der mindestens einen Abkühlzone mit der Nennleistung zum Ausbringen von Behandlungsmedium aus den Spritzvorrichtungen der mindestens einen Aufwärmzone und der mindestens einen Abkühlzone, und danach
- zu einem zweiten gegebenen Zeitpunkt vor einem geplanten Zuführen von Behältern (44) aus der mindestens einen Anwärmzone zu der mindestens einen Pasteurisierungszone, betreiben der Pumpe der mindestens einen Pasteurisierungszone mit der Nennleistung zum Ausbringen von Behandlungsmedium aus der Spritzvorrichtung der mindestens einen Pasteurisierungszone.

16. Das Verfahren nach Anspruch 15, weiter umfassend, zum Erreichen eines Stand-By-Betriebs der thermischen Behandlungsanlage nach Erreichen des Normalbetriebs der thermischen Behandlungsanlage:
- nachdem Behälter (44) die mindestens eine Pasteurisierungszone verlassen haben, Nicht-Betreiben der Pumpe der mindestens einen Pasteurisierungszone oder Betreiben der Pumpe der mindestens einen Pasteurisierungszone mit einer reduzierten Nennleistung zum Nicht-Ausbringen von Behandlungsmedium mittels der Spritzvorrichtung der mindestens einen Pasteurisierungszone, und danach
- nachdem Behälter (44) die letzte der mindestens einen Abkühlzone verlassen haben, Nicht-Betreiben aller Pumpen der mindestens einen Aufwärmzone und der mindestens einen Abkühlzone oder Betreiben aller Pumpen der mindestens einen Aufwärmzone und der mindestens einen Abkühlzone mit einer reduzierten Nennleistung zum Nicht-Ausbringen von Behandlungsmedium mittels der jeweiligen Spritzvorrichtungen.
